# EUROPÄISCHE PATENTSCHRIFT

(11) **EP 0 804 135 B1**
(45) Veröffentlichungstag und Bekanntmachung des Hinweises auf die Patenterteilung: **04.07.2001**
(21) Anmeldenummer: 96900966.1
(22) Anmeldetag: 18.01.1996
(51) Int. Cl.: A61K 6/00

(54) **ELASTISCH-PLASTISCHES ELEMENT ZUM FÜLLEN VON WURZELKANÄLEN UND HERSTELLUNGSVERFAHREN**
ELASTIC-PLASTIC ELEMENT FOR FILLING ROOT CANALS AND A METHOD OF MANUFACTURING THE SAID MATERIAL
ELEMENT PLASTIQUE ELASTIQUE DESTINE A REMPLIR DES CANAUX RADICULAIRES ET PROCEDE DE FABRICATION DUDIT ELEMENT

(30) Priorität: 18.01.1995 DE 19501374
(43) Veröffentlichungstag der Anmeldung: 05.11.1997
(73) Patentinhaber: ROEKO GmbH + Co. Dentalerzeugnisse, 89129 Langenau (DE)
(72) Erfinder: MANNSCHEDEL, Werner, D-89129 Langenau (DE)
(74) Vertreter: Boeters, Hans Dietrich, Dr.
(86) Internationale Anmeldenummer: EP9600192
(87) Internationale Veröffentlichungsnummer: WO9622069

(56) Entgegenhaltungen:
- DE-A- 3 628 823
- GB-A- 1 060 275
- US-A- 4 483 679
- US-A- 4 657 592

## Beschreibung

Die vorliegende Erfindung betrifft ein elastisch-plastisches Element mit einer Matrix auf Isopren-Basis und mit mindestens einem Füllstoff (Calciumsilikat ausgenommen), wobei das Element zum Füllen von Wurzelkanälen des Menschen oder eines Tieres geeignet ist. Sie betrifft ferner ein Herstellungsverfahren für das elastich-plastische Element.

Zur Therapie der als Pulpitis bekannten Erkrankung wird die erkrankte Pulpa mechanisch aus dem Wurzelkanal entfernt, der Wurzelkanal gereinigt und ausgebohrt, mit einem elastisch-plastischen Element oder einem anderen Füllmaterial gefüllt und danach versiegelt. Zum Stand der Technik sei beispielsweise auf Friedman et al. in J. Dent. Res., 54 (1975) 921-925, Briseno in Philipp J., 2, 90, 65-73 und US-A-4 632 977 verwiesen. Briseno beschreibt als Wurzelkanalfüllmaterialien unter anderem halbfeste Zemente auf Kunstharzbasis, Zinkoxid-Eugenol-Basis und Calziumhydroxidbasis. Die US-A-4 632 977 schlägt Füllmaterialien auf Trans-Poly-Isopren-Basis vor, beispielsweise auf Basis von Guttapercha oder Balata. Im Handel sind Guttapercha-Spitzen erhältlich, deren Standardzusammensetzung 20 Gew.-% Guttapercha als Matrix, 60 bis 75 Gew.-% Zinkoxid als Füllstoff, 1 bis 17 Gew.-% Schwermetallsulfate als Röntgenkontrastmittel und 3 bis 4 Gew.-% Wachse und Harze als Weichmacher vorsieht. Dieses bekannte Füllmaterial ist im Wurzelkanal inert und reagiert demgemäß nicht mit dem Körpergewebe.

Bei der Füllung eines Wurzelkanals mit einem bekannten inerten Füllmaterial ist es jedoch nicht ausgeschlossen, daß es beispielsweise durch im Kanal verbliebene Keime nach der Füllung langsam zu einem entzündlichen Prozeß kommt, der eine erneute Behandlung erforderlich macht, die häufig zum vollständigen Zahnverlust führt.

Gegenüber diesem Stand der Technik ist es Aufgabe der vorliegenden Erfindung, ein elastisch-plastisches Element zum Füllen von Wurzelkanälen des Menschen oder eines Tieres vorzusehen, das leicht und einfach hergestellt und verarbeitet werden kann und nicht mit den angesprochenen negativen Wirkungen belastet ist. Ferner ist es Aufgabe der vorliegenden Erfindung, ein Verfahren zur Herstellung eines derartigen Elements vorzusehen.

Diese Aufgabe wird gemäß einer Ausführungsform der Erfindung durch ein elastisch-plastisches Element mit einer Matrix auf Isopren-Basis und mit mindestens einem Füllstoff (Calciumsilikat ausgenommen) gelöst, wobei das Element zum Füllen von Wurzelkanälen des Menschen oder eines Tieres geeignet ist, ***gekennzeichnet*** durch in die Matrix eingearbeitetes Calciumhydroxid als Füllstoff sowie durch
(a) 43,5 bis 65,0 Gew.-% Matrix,
(b) 35,0 bis 56,5 Gew.-% Füllstoff,
(c) gegebenenfalls zusätzlich bis zu 8 Gew.-% Röntgenkontrastmittel, bezogen auf (a) und (b), und
(d) gegebenenfalls zusätzliche übliche Komponenten.

Calciumhydroxid läßt sich mit einer Matrix auf Isopren-Basis, wie Guttapercha, einfach verarbeiten, wobei es sich vorteilhaft als Pulver einwalzen läßt, was den Herstellungsprozeß wesentlich vereinfacht.

Da Calciumhydroxid alkalisch wirkt, kann es die sich bei entzündlichen Prozessen bildenden Säuren abpuffern und einen Anstieg des pH-Wertes eingrenzen. Dadurch wird der Heilungsprozeß vorteilhaft beschleunigt und unterstützt. Die Einlage kann dabei temporär oder über einen längeren Zeitraum im Wurzelkanal verbleiben.

Dokument DE-A-3 628 823 beschreibt ein Element mit einer Matrix auf Guttapercha-Basis, in die Calciumhydroxid und Calciumsilicat als Füllstoffe eingearbeitet sind. Das Element ist zum Füllen von Wurzelkanälen geeignet.

In der US-A-4 657 592 wird eine Dichtungsmasse für Wurzelkanäle offenbart, die zusammen mit einer Guttapercha-Spitze in einen Wurzelkanal eingebracht wird.

Das Dokument GB-A-1 060 275 betrifft einen Dentalzement.

Erfindungsgemäß kann eine Matrix auf Trans-Poly-Isopren-Basis, auf Guttapercha-Basis oder auf Balata-Basis vorgesehen werden. Die Matrix kann dabei durch mindestens 80 Gew.-% Trans-Poly-Isopren gekennzeichnet sein.

Das erfindungsgemäße elastisch-plastische Element kann in Form einer Guttapercha-Spitze vorliegen.

Das erfindungsgemäß als Füllstoff eingesetzte Calciumhydroxid kann in Form eines Gemischs mit einem üblichen anderen Füllstoff, wie Zinkoxid, oder als alleiniger Füllstoff vorgesehen werden.

Selbstverständlich kann das erfindungsgemäße elastisch-plastische Element zusätzlich einen pharmazeutischen Wirkstoff, insbesondere einen in wässerigem Medium lösbaren dispergierbaren Wirkstoff, beispielsweise ein Antibiotikum und/oder Glucocorticoid, und ferner übliche Wachse, Harze oder sonstige Hilfsstoffe umfassen.

So kann das erfindungsgemäße elastisch-plastische Element beispielsweise durch
(a) etwa 43,5 Gew.-% Guttapercha,
(b) etwa 56,5 Gew.-% Calciumhydroxid als alleinigen anorganischen Füllstoff,
(c) gegebenenfalls zusätzlich bis zu 8 Gew.-% Röntgenkontrastmittel, bezogen auf (a) und (b), und
(d) gegebenenfalls zusätzliche übliche Komponenten gekennzeichnet sein.

Das erfindungsgemäße elastisch-plastische Element weist damit im wesentlichen drei Komponenten auf, die im folgenden noch näher erläutert werden sollen.

### Komponente 1

Die mechanischen Eigenschaften eines erfindungsgemäßen elastisch-plastischen Elements werden in erster Linie durch die Eigenschaften der Matrix bestimmt, die 43,5 bis 65,0 Gew.-% neben 35,0 bis 56,5 Gew.-% Füllstoff (Komponente 2) ausmacht. Die Matrix muß einerseits elastisch sein, damit sie bequem verarbeitet und leicht in den Wurzelkanal eingebracht werden kann. Andererseits soll sie plastische Eigenschaften aufweisen, damit der Wurzelkanal dauerhaft ausgefüllt werden kann, ohne daß ein Wandspalt verbleibt. Darüberhinaus muß die Matrix andere Komponenten, insbesondere den Füllstoff, leicht aufnehmen können. Für diese Zwecke hat sich eine Matrix mit mindestens 80 Gew.-% Trans-Poly-Isopen als vorteilhaft erwiesen. Als Beispiel kann Guttapercha angeführt werden, bei dem es sich um eine Matrix auf natürlicher Basis handelt, deren Hauptbestandteil Trans-Poly-Isopren ist. Selbstverständlich sind auch andere Trans-Poly-Isoprene einsetzbar, wie Balata, synthetische Matrizes auf Isopren-Basis oder Abkömmlinge der genannten Materialien.

### Komponente 2

Das erfindungsgemäße elastisch-plastische Element ist durch Calciumhydroxid als Füllstoff gekennzeichnet. Dieser Füllstoff liegt in einer Menge von 35,0 bis 56,5 Gew.-% neben 43,5 bis 65,0 Gew.-% Matrix vor. Bei Calciumhydroxid handelt es sich um einen gewebe- bzw. körperverträglichen Stoff, der mit der Matrix gemischt und zusätzlich mit pharmazeutischen Wirkstoffen sowie üblichen Hilfsmitteln compoundiert werden kann.

Calciumhydroxid läßt sich als Pulver in Guttapercha einwalzen, was den Herstellungsprozeß recht einfach gestalten läßt.

Wie bereits gesagt, kann Calciumhydroxid den bei entzündlichen Prozessen üblicherweise auftretenden pH-Anstieg abpuffern. Es ist daher erwünscht, möglichst viel Calciumhydroxid in den Wurzelkanal einzubringen. Der Anteil an Calciumhydroxid im erfindungsgemäßen elastisch-plastischen Element orientiert sich daher einerseits an diesem Wunsch und andererseits am Aufnahmevermögen der Matrix für den organischen Füllstoff. Ein Beispiel für ein elastisch-plastisches Element mit einem recht hohen Füllstoff-Anteil ist eine Guttapercha-Spitze mit einem Gehalt von etwa 56,5 Gew.-% Calciumhydroxid neben etwa 43,5 Gew.-% Guttapercha.

### Komponente 3

Als weitere, allerdings fakultative, wenn auch übliche Komponente kann ein Röntgenkontrastmittel vorgesehen werden, beispielsweise ein Schwermetallsulfat, wie Bariumsulfat, oder eine Wismut-Strontium-Verbindung. Diese Komponente kann in einer Menge von bis zu 8 Gew.-% auf Basis von Matrix und Füllstoff vorgesehen werden.

Gemäß einer weiteren Ausführungsform der Erfindung wird schließlich ein Verfahren zum Herstellen eines erfindungsgemäßen elastisch-plastischen Elements vorgesehen, das dadurch gekennzeichnet ist, daß man
(a) einen Film einer Matrix auf Isopren-Basis bildet,
(b) in den Film der Matrix mindestens einen Füllstoff sowie gegebenenfalls ein Röntgenkontrastmittel und weitere fakultative übliche Hilfsmittel vorzugsweise in Pulverform oder nicht zu hoch viskoser Form einwalzt,
(c) den compoundierten Film zerkleinert und extrudiert,
(d) das Extrudat zerschneidet und den anfallenden Schnitt zu elastisch-plastischen Elementen rollt, die zum Füllen von Wurzelkanälen des Menschen oder eines Tieres geeignet sind. Nachstehend wird die Erfindung durch ein Beispiel näher erläutert.

### Beispiel

Zur Herstellung von Guttaperchaspitzen wurde Guttapercha als Matrix zunächst zwischen einer Trägerwalze und einer Andruckwalze verwalzt, so daß sich die Guttapercha als dünner Film bzw. fellartig um die Trägerwalze legte. Danach wurde pulverförmiges Calciumhydroxid in einem Gewichtsverhältnis von 56,5 % Ca(OH)₂: 43,5 % Guttapercha in den gebildeten Film eingewalzt, der Film von der Trägerwalze abgeschält, der abgeschälte Film zerkleinert und zu einem dünnen drahtartigen Strang extrudiert. Dieser Strang wurde danach zerschnitten und rolliert.

Die gebildeten elastisch-plastischen Elemente ließen sich leicht in Wurzelkanäle einführen und legten sich ohne Kanalbildung an.

## Patentansprüche

1. Elastisch-plastisches Element mit einer Matrix auf Isopren-Basis und mit mindestens einem Füllstoff (Calciumsilikat ausgenommen), wobei das Element zum Füllen von Wurzelkanälen des Menschen oder eines Tieres geeignet ist, ***gekennzeichnet*** durch in die Matrix eingearbeitetes Calciumhydroxid als Füllstoff sowie durch
(a) 43,5 bis 65,0 Gew.-% Matrix,
(b) 35,0 bis 56,5 Gew.-% Füllstoff,
(c) gegebenenfalls zusätzlich bis zu 8 Gew.-% Röntgenkontrastmittel, bezogen auf (a) und (b), und
(d) gegebenenfalls zusätzliche übliche Komponenten.

2. Element nach Anspruch 1, ***gekennzeichnet*** durch eine Matrix auf Trans-Poly-Isopren-Basis, Guttapercha-Basis oder Balata-Basis.

3. Element nach Anspruch 1 oder 2, ***gekennzeichnet*** durch eine Matrix mit mindestens 80 Gew.-% Trans-Poly-Isopren.

4. Element nach einem der vorhergehenden Ansprüche in Form einer Guttapercha-Spitze.

5. Element nach einem der vorhergehenden Ansprüche, ***gekennzeichnet*** durch ein Zinkoxid/Calciumhydroxid-Gemisch oder durch Zinkoxid-freies Calciumhydroxid als Füllstoff.

6. Element nach einem der vorhergehenden Ansprüche, ***gekennzeichnet*** durch einen zusätzlichen Gehalt an einem üblichen pharmazeutischen Wirkstoff, insbesondere einen in wässerigem Medium lösbaren oder dispergierbaren Wirkstoff.

7. Element nach Anspruch 6, ***gekennzeichnet*** durch ein Antibiotikum und/oder Glucocorticoid als pharmazeutischer Wirkstoff.

8. Element nach einem der vorhergehenden Ansprüche, ***gekennzeichnet*** durch einen zusätzlichen Gehalt an einem üblichen Wachs und/oder Harz.

9. Element nach einem der vorhergehenden Ansprüche, ***gekennzeichnet*** durch
(a) etwa 43,5 Gew.-% Guttapercha,
(b) etwa 56,5 Gew.-% Calciumhydroxid als alleinigen anorganischen Füllstoff,
(c) gegebenenfalls zusätzlich bis zu 8 Gew.-% Röntgenkontrastmittel, bezogen auf (a) und (b), und
(d) gegebenenfalls zusätzliche übliche Komponenten.

10. Verfahren zum Herstellen eines elastisch-plastischen Elements gemäß einem der vohergehenden Ansprüche, dadurch ***gekennzeichnet***, daß man
(a) einen Film einer Matrix auf Isopren-Basis bildet,
(b) in den Film der Matrix mindestens einen Füllstoff sowie gegebenenfalls ein Röntgenkontrastmittel und weitere fakultative übliche Hilfsmittel einwalzt,
(c) den kompoundierten Film zerkleinert und extrudiert,
(d) das Extrudat zerschneidet und den anfallenden Schnitt zu elastisch-plastischen Elementen rollt, die zum Füllen von Wurzelkanälen des Menschen oder eines Tieres geeignet sind.

## Claims

1. Elastic-plastic element comprising an isoprene-based matrix and at least one filler (calcium silicate being excepted), the element being suitable for filling root canals of humans or animals, **characterised** by calcium hydroxide, incorporated into the matrix, as filler, and by
(a) from 43.5 to 65.0% by weight of matrix,
(b) from 35.0 to 56.5% by weight of filler,
(c) optionally additionally up to 8% by weight of X-ray contrast agent, based on (a) and (b), and
(d) optionally additional customary components.

2. Element according to claim 1, **characterised** by a matrix based on trans-polyisoprene, gutta-percha or on balata.

3. Element according to claim 1 or claim 2, **characterised** by a matrix containing at least 80% by weight of trans-polyisoprene.

4. Element according to any one of the preceding claims in the form of a guttapercha point.

5. Element according to any one of the preceding claims, **characterised** by a zinc oxide/calcium hydroxide mixture or by zinc oxide-free calcium hydroxide as filler.

6. Element according to any one of the preceding claims, **characterised** in that it additionally comprises a customary pharmaceutical active ingredient, especially an active ingredient that is soluble or dispersible in an aqueous medium.

7. Element according to claim 6, **characterised** by an antibiotic and/or glucocorticoid as pharmaceutical active ingredient.

8. Element according to any one of the preceding claims, **characterised** in that it additionally comprises a customary wax and/or resin.

9. Element according to any one of the preceding claims, **characterised** by
(a) approximately 43.5% by weight of gutta-percha,
(b) approximately 56.5% by weight of calcium hydroxide as sole inorganic filler,
(c) optionally additionally up to 8% by weight of X-ray contrast agent, based on (a) and (b), and
(d) optionally additional customary components.

10. Method of preparing an elastic-plastic element according to any one of the preceding claims, **characterised** by
(a) forming a film of an isoprene-based matrix;
(b) rolling into the matrix film at least one filler and optionally an X-ray contrast agent and further optional customary excipients;
(c) comminuting and extruding the compounded film;
(d) cutting up the extruded material and rolling the cut piece to form elastic-plastic elements that are suitable for filling root canals of humans or animals.

## Revendications

1. Élément élastique-plastique comprenant une matrice à base d'isoprène et au moins une charge (à l'exclusion du silicate de calcium), ledit élément convenant au remplissage des canaux radiculaires chez l'homme ou chez l'animal,
caractérisé en ce que de l'hydroxyde de calcium est intégré à titre de charge dans la matrice, et ce que :
(a) la matrice représente 43,5 à 65,0 % en poids,
(b) la charge représente 35,0 à 56,5 % en poids,
(c) il est prévu le cas échéant en supplément jusqu'à 8 % en poids d'agent de contraste aux rayons X, rapporté à (a) et (b), et
(d) il est prévu le cas échéant des composants habituels additionnels.

2. Élément selon la revendication 1, caractérisé par une matrice à base de trans-poly-isoprène, à base de gutta-percha, ou à base de balata.

3. Élément selon l'une ou l'autre des revendications 1 et 2, caractérisé par une matrice comprenant au moins 80 % en poids de trans-poly-isoprène.

4. Élément selon l'une des revendications précédentes, sous la forme d'une pointe en gutta-percha.

5. Élément selon l'une des revendications précédentes, caractérisé par un mélange oxyde de zinc/hydroxyde de calcium, ou par de l'hydroxyde de calcium dépourvu d'oxyde de zinc, à titre de charge.

6. Élément selon l'une des revendications précédentes, caractérisé par une teneur supplémentaire d'un produit actif pharmaceutique habituel, en particulier un produit actif soluble ou dispersible dans un milieu aqueux.

7. Élément selon la revendication 6, caractérisé par un antibiotique et/ou un glucocorticoïde à titre de produit actif pharmaceutique.

8. Élément selon l'une des revendications précédentes, caractérisé par une teneur en supplémentaire d'une cire et/ou d'une résine habituelle.

9. Élément selon l'une des revendications précédentes, caractérisé par :
(a) environ 43,5 % en poids de gutta-percha,
(b) environ 56,5 % en poids d'hydroxyde de calcium à titre de charge inorganique unique,
(c) le cas échéant en supplément jusqu'à 8 % en poids d'agent de contraste aux rayons X, rapporté à (a) et (b), et
(d) le cas échéant des composants habituels supplémentaires.

10. Procédé pour produire un élément élastique-plastique selon l'une des revendications précédentes, caractérisé en ce que :
(a) on forme un film d'une matrice à base d'isoprène,
(b) on incorpore par laminage dans le film de la matrice au moins une charge, ainsi que le cas échéant un agent de contraste aux rayons X et d'autres agents auxiliaires habituels facultatifs,
(c) on broie le film composite et on l'extrude,
(d) on découpe le produit extrudé et l'on roule les morceaux découpés résultants en éléments élastiques-plastiques qui conviennent au remplissage des canaux radiculaires chez l'homme ou chez l'animal.
